# EUROPEAN PATENT APPLICATION

(11) **EP 4 129 252 A1**
(43) Date of publication of application: **08.02.2023**
(21) Application number: 20928974.3
(22) Date of filing: 22.10.2020
(51) Int. Cl.: A61F 6/04, C08J 5/12, B29C 41/14

(54) **POLYURETHANE CONDOM WITH ADHESIVE LAYER AND PREPARATION METHOD THEREFOR**

(30) Priority: 31.03.2020 CN 202010242233; 20.05.2020 CN 202010430499; 20.05.2020 CN 202010431395
(71) Applicant: Reckitt Benckiser Health Limited, Slough, Berkshire SL1 3UH (GB)
(72) Inventor: DAI, Jiabing, Lanzhou, Gansu 730000 (CN); LI, Weihu, Lanzhou, Gansu 730000 (CN); FENG, Linlin, Lanzhou, Gansu 730000 (CN); CHEN, Liang, Lanzhou, Gansu 730000 (CN)
(74) Representative: O'Brien, Niall James
(86) International application number: PCT/CN2020/122617
(87) International publication number: WO 2021/196570

(57) **Abstract**

Disclosed are a polyurethane condom (100) with an adhesive layer (20) and a preparation method therefor. The polyurethane condom (100) with the adhesive layer (20) comprises a first polyurethane film layer (10), the adhesive layer (20) on the first polyurethane film layer (10), and a second film layer (30) on the adhesive layer (20), wherein the adhesive layer (20) includes 0 to 100% by weight of a polar component, 0 to 100% by weight of a non-polar component, and 0 to 100% by weight of a material composed of a polar and non-polar hybrid component, and has a thickness of 0.1 to 30 µm.

## Description

### Technical Field

The present disclosure relates to the field of condom materials, and in particular, to a polyurethane condom having an adhesive layer and a method for preparing the condom.

### Background

Condoms are simple devices widely used in the world for contraception and prevention of sexually transmitted diseases. The current condom products mainly include natural rubber latex condoms and waterborne polyurethane condoms.

At present, natural rubber latex condoms are thick because of the material characteristics of natural latex. Meanwhile, natural latex contains a variety of proteins, which pose a safety risk to people having an allergy to proteins. In addition, polyisoprene as a condom material has soft texture, good transparency, and no protein, but it also has problems of a large thickness and low strength.

With the improvement of living standards, consumers of condoms not only require the basic contraception function, but also demand a better user experience. Polyurethane condoms, as a new product in the market in recent years, provide consumers with a more realistic and pleasant experience during use due to the high strength and high biosafety of the material. However, the flexibility of polyurethane materials is poor compared with natural rubbers or polyisoprene, and it is difficult for a single polyurethane resin to be ideal in flexibility, burst property, water resistance, and strength. In addition, even if polyurethane and natural latex or polyisoprene are made into a composite, since polyurethane resin is a polar material while natural rubber and polyisoprene are non-polar materials, poor adhesion occurs due to the different polarities of the materials in the composite, which may cause delamination and thus prevent industrial-scale production.

Therefore, it is of great significance to provide a new condom product having an optimal performance and meeting the market demands.

### Summary of the invention

The present invention is made in view of the above-described drawbacks in the prior art. One of the objectives of the present disclosure is to provide a polyurethane condom with an adhesive layer, which has very small thickness and excellent flexibility and strength meeting the market demands.

The present disclosure provides a polyurethane condom having an adhesive layer, comprising at least: a first polyurethane film layer; an adhesive layer on the first polyurethane film layer; and a second film layer on the adhesive layer, wherein the adhesive layer includes 0 to 100% by weight of a polar component, 0 to 100% by weight of a non-polar component, and 0 to 100% by weight of a material composed of a polar and non-polar hybrid component, and has a thickness of 0.1 to 30 µm.

In one embodiment, the polyurethane condom having an adhesive layer has a burst pressure of 1 kPa or more, and/or a burst volume of 5 L or more.

In one embodiment, the polyurethane condom having an adhesive layer has a thickness of 0.010 to 0.080 mm.

In one embodiment, the adhesive layer has a 100% tensile modulus of 2.5 N/mm² or lower.

In one embodiment, the polar component is any one selected from polyurethane resins, neoprene, polyvinyl acetate resins, acrylonitrile-butadiene-styrene, vinyl acetate-ethylene copolymer resins, polyacrylates, polyvinyl chlorides, proteins, starches, celluloses, and epoxy resins, or any combination thereof.

In one embodiment, the adhesive layer includes 100% by weight of the polar component which is a polyurethane resin.

In one embodiment, the polyurethane resin is any one selected from anionic, cationic, nonionic, and amphoteric waterborne polyurethanes, or solvent-based and solvent-free polyurethanes, or any combination thereof.

In one embodiment, the polyurethane resin is any one of polyether-based, polyester-based, and polyether-polyester hybrid polyurethane resins, or any combination thereof.

In one embodiment, the anionic waterborne polyurethane is a carboxylic acid-based waterborne polyurethane, a sulfonic acid-based waterborne polyurethane, or a mixture of both.

In one embodiment, the non-polar component is one selected from natural rubbers, polyolefins, silicone resins, butyl rubbers, styrene-butadiene rubbers, ethylene-propylene-diene rubbers, polystyrenes, polyisoprenes, polyesters, rosin resins, terpene resins, rosin resins and stretched forms thereof, or any combination thereof.

In one embodiment, the polar and non-polar hybrid component is selected from organically modified polar or non-polar polymeric materials.

In one embodiment, the organic modification is any one selected from organosilicon modification, organofluorine modification, silane modification, acrylate copolymerization modification, vinyl modification, aromatic monomer modification, polyether modification, polyester modification, isocyanate modification, amination, carboxylation, hydroxylation, epoxidation, sulfonation, and polymerization modification, or any combination thereof.

In one embodiment, the adhesive layer includes 50% by weight of a polar component and 50% by weight of a non-polar component.

In one embodiment, there is at least one adhesive layer.

In one embodiment, the second film layer has a 100% modulus of 1 N/mm² or lower.

In one embodiment, the second film layer is a layer composed of a polyurethane resin.

In one embodiment, the second film layer is a layer composed of a natural rubber or a polyisoprene resin.

The present disclosure also provides a polyurethane condom having an adhesive layer, comprising: a first polyurethane film layer; a first adhesive layer on the first polyurethane film layer; a second film layer on the adhesive layer; a second adhesive layer on the second film layer, having the same structure as that of the first adhesive layer; a third polyurethane film layer on the second film layer, having the same structure as that of the first polyurethane film layer, wherein the first adhesive layer includes 0 to 100% by weight of a polar component, 0 to 100% by weight of a non-polar component, and 0 to 100% by weight of a material composed of polar and non-polar hybrid component, and has a thickness of 0.1-30 µm and a 100% tensile modulus of 2.5 N/mm² or lower; the second film layer includes a natural rubber or a polyisoprene resin; and the polyurethane condom has a thickness of 0.010-0.080 mm.

The present disclosure further provides a method for preparing the polyurethane condom having an adhesive layer, comprising at least the steps of:
- dipping in a polyurethane resin and drying, to form a first polyurethane film layer;
- dipping in an adhesive layer material and drying, to form an adhesive layer on the first polyurethane film layer;
- dipping in a second film layer material and drying, to form a second film layer on the adhesive layer;
- dipping in a release agent to apply it on the second film layer, followed by drying, demolding, and electrical testing to obtain the polyurethane condom;
wherein the adhesive layer material includes 0 to 100% by weight of a polar component, 0 to 100% by weight of a non-polar component, and 0 to 100% by weight of a material composed of polar and non-polar hybrid component, and has a thickness of 0.1-30 µm.

In one embodiment, the dipping in a first polyurethane resin is carried out by dipping a mould composed of glass or a thermoplastic polymer in the first polyurethane resin.

The polyurethane condom having an adhesive layer provided according to the present disclosure employs an adhesive layer sandwiched within a polyurethane condom, thereby bonding film layers having the same property or different properties on the opposite sides of the adhesive layer together to make a composite article, and making full use of the respective performance advantages of the material of each layer. The condom has a thickness of 0.010-0.080 mm, and has excellent properties such as flexibility and strength, so as to meet the market demand for the product. In addition, the polyurethane condom having an adhesive layer has optimized interlayer adhesion in that no delamination is observed when it is immersed in an alkali solution at 50°C to 70°C for 1-10 min. In addition, the present disclosure uses a formulation having a low cost and employs dip moulding which is a simple, safe, and environmentally friendly moulding process. Other features, benefits, and advantages will be apparent from the disclosure detailed herein, including the description and claims.

### Brief Description of the Drawings

The drawings used in the embodiments will be briefly described below to more clearly describe the technical solutions in the embodiments of the present disclosure. It is to be understood that the following drawings depict only certain embodiments of the invention and are therefore not to be considered limiting its scope. For a person of ordinary skill in the art, other relevant drawings can also be obtained according to these drawings without paying any inventive effort.
Fig. 1 illustrates a schematic structure of a polyurethane condom having an adhesive layer according to one specific embodiment of the present disclosure;
Fig. 2 illustrates a schematic view of the layered structure of the polyurethane condom of Fig. 1;
Fig. 3 illustrates a schematic view of the layered structure of the polyurethane condom according to another specific embodiment;
Fig. 4 illustrates a schematic flow diagram of a method for preparing the polyurethane condom having an adhesive layer according to one specific embodiment of the present disclosure.

### Detailed Description of the Invention

The technical solutions of the embodiments in the present disclosure will be clearly and fully described with reference to the accompanying drawings, in order to provide a clear understanding of for the objectives, technical solutions and advantages of the embodiments of the present disclosure. Obviously, it is to be understood that the described embodiments are part of, and not all of, the present disclosure. The components of the embodiments in the present disclosure, which are generally described and illustrated in the drawings herein, can be configured and designed upon different needs.

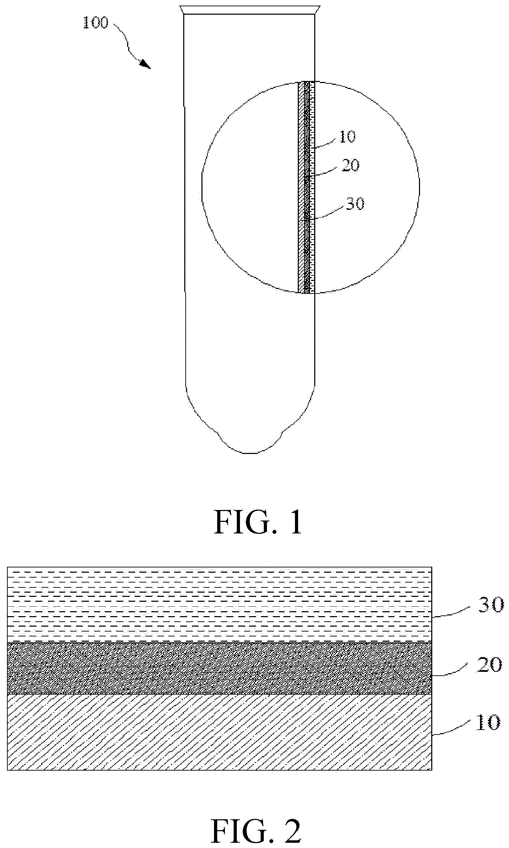

As shown in Figs. 1-4, the present disclosure provides a polyurethane condom 100, which is made of materials comprising polyurethane, has high strength, high compactness, and good biocompatibility, may have a thickness of for example 0.001 mm to 0.08 mm, preferably 0.05 mm to 0.04 mm, such as 0.005 mm, 0.01 mm, 0.015 mm, and 0.02 mm, and can provide a user with a more comfortable experience, and a softer and more delicate skin feel, without the problem of allergy. The polyurethane condom 100 provided herein shows no delamination when immersed in an alkali solution at 50°C to 70°C for 1-10 min, for example, when immersed in a sodium hydroxide solution at 50°C for 3 min.

The polyurethane condom 100 having a thickness within the above range has a burst pressure of for example 1 kPa or more, preferably 3 kPa or more, for example, 3 kPa, 4 kPa, or 5kPa, and a burst volume of for example 5 L or more, for example, 8 L, 12 L, or 18 L.

The polyurethane condom 100 may have a strength, e.g. tensile strength, of for example 30 MPa or more, preferably 40 MPa or more, and a 100% modulus of for example 2.5 N/mm² or lower, such as 2 N/mm², 1.8 N/mm², 1.5 N/mm², or 1 N/mm².

As shown in Figs. 1 and 2, the present disclosure provides a specific embodiment of the polyurethane condom 100, which may be composed of a multi-layer structure, such as 3-layer, 4-layer, 5-layer, 6-layer, etc., but not limited thereto, as adjustable according to actual needs. Here the polyurethane condom 100 is configured as a male condom, but it should be understood that it may also be configured as a female condom, or other heterotypic condoms and the like. Any polyurethane condom 100 product comprising the layered configuration of the present disclosure should fall within the scope of the present disclosure.

As shown in Figs. 1 and 2, the polyurethane condom 100 includes a first polyurethane film layer 10, an adhesive layer 20, and a second film layer 30. The first polyurethane film layer 10 may serve as the innermost layer of the polyurethane condom 100, contacting the skin during use; or may certainly serve as the outermost layer, as adjustable according to actual needs.

As shown in Fig. 2, the first polyurethane film layer 10 may be a layer formed of a polyurethane resin, or a layer formed of an emulsion of the polyurethane resin mixed with other auxiliary agents. The auxiliary agents may be, for example, functional auxiliary agents such as a lubricant, a bactericide, a thickener, a wetting agent, an antifoaming agent and the like, but not limited thereto. The polyurethane resin may be any suitable kind of aliphatic polyurethane or aromatic polyurethane, and may be preferably, for example, an anionic, cationic, nonionic, or amphoteric waterborne polyurethane, or may also be a solvent-based or solvent-free polyurethane. The raw materials and preparation process for the polyurethane may be adjusted to prepare a desired polyurethane.

In some embodiments, the polyurethane resin may have a tensile strength of for example 25 MPa or more, preferably 30 MPa or more, for example, 35 MPa, 38 MPa, 40 MPa, 55 MPa, 60 MPa or more, so as to form a high-strength polyurethane resin A, from which a first polyurethane film layer 10 formed has a high tensile strength and is not easily damaged.

In some embodiments, the polyurethane resin may have a 100% tensile modulus of, for example, 3.5N/mm² or lower, such as 3.2 N/mm², 3 N/mm², 2.8 N/mm², 2.5 N/mm², 2 N/mm², 1.8 N/mm², 1.5 N/mm² or 0.6 N/mm² or lower, to form a low-modulus polyurethane resin B, from which a first polyurethane film layer 10 formed has good flexibility.

In some embodiments, the polyurethane resin may have an elongation at break of, for example, 500% or more, for example, 600%, 700% or more, preferably 800% or more, for example 830%, 900%, 1000% or more, so as to form a high-ductility polyurethane resin C, from which a first polyurethane film layer 10 formed has high ductility and stretchability.

In some embodiments, the polyurethane resin has a wet-film tensile strength retention of 60% or more, for example, 65% or 75%, after a dry film formed therefrom is immersed in water at 30°C to 60 °C for 0.1-1h, such as in water at 40°C for 0.5 h, in water at 50°C for 0.5 h, or in water at 50°C for 0.6 h, thereby forming a water-resistant polyurethane resin D, from which a first polyurethane film layer 10 formed has good water resistance.

In some embodiments, the polyurethane resin may be used alone to make a condom, and such a condom of 10-25 µm, such as 20 µm or 22 µm, made thereof has a burst pressure of 1.0 kPa or more, for example 2.0 kPa, 3.0 kPa, 4.0 kPa, 5.0 kPa or more, thereby forming a high burst-pressure polyurethane resin E.

In some embodiments, the polyurethane resin may be used alone to make a condom, and such a condom of 10-25 µm, such as 20 µm or 22 µm, made thereof has a burst volume of 5 L or more, for example 8 kPa, 10 kPa, 12 kPa, 15 kPa, 18 kPa or more, thereby forming a high burst-volume polyurethane resin F.

It should be understood that only some properties of the polyurethane resin for the first polyurethane film layer 10 are described here, the properties of the polyurethane resin are certainly not limited thereto, and polyurethane resin with more properties may be achieved according to actual needs, such as tear resistance, oil resistance, blocking resistance, electric breakdown resistance, etc.

As shown in Fig. 2, the first polyurethane film layer 10 may have a thickness of for example 0.1-20 µm, such as 0.3 µm, 1 µm, 3 µm, 5 µm, 10 µm.

Referring to Figs. 1 and 2 again, the adhesive layer 20 is on the first polyurethane film layer 10, and may include 0 to 100% by weight of a polar component, 0 to 100% by weight of a non-polar component, and 0 to 100% by weight of a material composed of polar and non-polar hybrid component. For example, the adhesive layer 20 is a layer composed of 100% by weight of a polar component showing philicity toward polyurethane, or a layer composed of 100% by weight of a non-polar component showing philicity toward a natural rubber or polyisoprene resin, or a layer composed of 100% by weight of a polar and non-polar hybrid component, or a layer composed of 50% by weight of the polar component and 50% by weight of the non-polar component, or a layer composed of 50% by weight of the polar component, 30% by weight of the non-polar component, and 20% by weight of the polar and non-polar hybrid component, etc.

The polar component is, for example, a polymer material having a polar group, and examples thereof may include polyurethane, neoprene, polyvinyl acetate resins, acrylonitrile-butadiene-styrene, vinyl acetate-ethylene copolymer resins, polyacrylates, polyvinyl chlorides, proteins, starches, cellulose, epoxy resins, etc. For example, the adhesive layer 20 may be a layer formed of 100% by weight of a polyurethane resin, or a layer formed of an emulsion of the polyurethane resin mixed with other auxiliary agents. The auxiliary agents may be, for example, functional auxiliary agents such as a lubricant, a bactericide, a thickener, a wetting agent, an antifoaming agent and the like, but certainly not limited thereto. The polyurethane resin may be any suitable kind of aliphatic polyurethane or aromatic polyurethane, and preferably, for example, an anionic, cationic, nonionic, amphoteric, solvent-based or solvent-free polyurethane. More preferably, the anionic polyurethane is carboxylic acid-based, sulfonic acid-based, or a mixture of both. The raw materials and preparation process for the polyurethane may be adjusted to prepare a desired polyurethane. From the viewpoints of obtaining the adhesion of the adhesive layer 20 and balancing the properties between the first polyurethane film layer 10 and the second film layer 30, the polyurethane resin for the polyurethane adhesive layer 20 is different from the polyurethane resins for the other film layers, and the polyurethane resin is a low-modulus polyurethane material synthesized from a polyether and/or polyester polyol. Since a polyether-based, polyester-based, or polyether-polyester hybrid polyurethane resin is obtained, the polyurethane resin has a good adhesiveness to other materials such as polyurethane and natural rubbers or polyisoprenes while having the characteristics of flexibility.

The non-polar component is, for example, a polymer material having a non-polar group, and examples thereof may include natural rubbers, polyolefins, silicone resins, butyl rubbers, styrene-butadiene rubbers, ethylene-propylene-diene rubbers, polystyrenes, polyisoprenes, polyesters, rosin resins, terpene resins, rosin resins and stretched forms thereof.

The polar and non-polar hybrid component refers to, for example, a polymer material having both a polar group and a non-polar group so as to exhibit both polarity and non-polarity. It can be obtained by modifying a polar material, such as a polar polymer material, with a material having a non-polar group. In some specific embodiments, a waterborne polyurethane is modified with a polyisoprene polyol or a vinyl group. It is certainly also possible that a non-polar material, such as a non-polar polymer material, is modified with a material having a polar group. For example, a natural rubber may be modified with a polyvinyl alcohol or a polyacrylic acid, thereby obtaining a polymer material having both a polar group and a non-polar group.

Furthermore, it should also be understood that the modification process may be carried out by organic modification of the polar or non-polar material to obtain a polymeric material having both polar and non-polar groups. The organic modification includes, without limitation, organosilicon modification, organofluorine modification, silane modification, acrylate copolymerization modification, vinyl modification, aromatic monomer modification, polyether modification, amination, carboxylation, epoxidation, hydroxylation, sulfonation, or polymerization modification. Furthermore, the modification may be chemical or physical modification, or modification in any other suitable manners.

In other embodiments, the material for the adhesive layer 20 may be reactive with the first polyurethane film layer 10 or the second film layer 30. Specifically, the material for the adhesive layer 20 has for example a vinyl group, a silane group, an amino group, a carboxyl group, or a hydroxyl group, allowing reaction with polyurethane or a vinyl group, so that the adhesive layer 20 can form a chemical bond with polyurethane or natural rubber.

The adhesive layer 20 has a structure different from the other film layers. The material for the adhesive layer 20 has a 100% tensile modulus of 2.5 N/mm² or lower, such as 2 N/mm², 1.8 N/mm², 1.5 N/mm², 1.2 N/mm², 1 N/mm², or 0.8 N/mm².

Referring to Figs. 1 and 2 again, the adhesive layer 20 is an intermediate layer which tightly bonds the film layers on its opposite sides. There may be at least one adhesive layer, e.g. one layer, two layers, three layers, etc. with thin film layers of other materials laminated on both sides of each adhesive layer. The adhesive layer 20 may have a thickness of for example 0.1-30 µm, preferably 0.1-20 µm, such as 1 µm, 2 µm, 3 µm, 5 µm, 10 µm, or 15 µm, in view of obtaining a low-modulus, highly adhesive adhesive layer 20 and a polyurethane condom 100 having desired performance. In addition, since the material for the adhesive layer 20 has a low modulus and a low strength, the ratio of the thickness of the adhesive layer 20 to the total thickness of the polyurethane condom 100 is 40% or less, and preferably 35% or less, such as 30% or 25%.

As shown in Fig. 2, a second film layer 30 is on the adhesive layer 20, and the second film layer 30 may be composed of, for example, any material that can form a film.

As shown in Fig. 2, the second film layer 30 may have a thickness of for example 0.1-30 µm, such as 1 µm, 2 µm, 3 µm, 5 µm, 10 µm, 15 µm, or 20 µm, in view of obtaining a polyurethane condom 100 having a desired thickness and desired interlayer adhesion.

In some embodiment, for example, the second film layer 30 is a layer formed of a polyurethane resin, or a layer formed of an emulsion of the polyurethane resin mixed with other auxiliary agents. The auxiliary agent may be, for example, functional auxiliary agents such as a lubricant, a bactericide, a thickener, a wetting agent, or an antifoaming agent, but certainly not limited thereto.

Herein, the second film layer 30 may have a structure, for example, the same as that of the first polyurethane film layer 10. Specifically, for example, the polyurethane resin for the second polyurethane film layer 30 has the same properties as those of the first polyurethane film layer 10. Specifically, for example, the first polyurethane film layer 10 and the second polyurethane film layer 30 are of the same polyurethane resin, both being the high-strength polyurethane resin A, or both being the low-modulus polyurethane resin B, or both being the high-ductility polyurethane resin C, and are bonded via the adhesive layer 20 (for example, the adhesive layer resin is represented by "N"), thereby obtaining a high-strength ANA-layer-structure polyurethane condom 100, or a flexible BNB-layer-structure polyurethane condom 100, or a high-ductility CNC-layer-structure polyurethane condom 100, respectively. In addition, for example, the second polyurethane film layer 30 and the first polyurethane film layer 10 may have the same thickness, for example, a thickness of 10 µm, 15 µm, or 20 µm. The present invention is certainly not limited thereto.

The second film layer 30 may have a structure for example different from that of the first polyurethane film layer 10. Specifically, for example, the polyurethane resin for the second polyurethane film layer 30 has properties different from those of the first polyurethane film layer 10. Specifically, for example, the polyurethane resin for the first polyurethane film layer 10 is a high-strength polyurethane resin A, and the polyurethane resin for the second polyurethane film layer 30 is a low-modulus polyurethane resin B, and they are bonded via the adhesive layer 20 to obtain a polyurethane condom 100 having an ANB layer structure having both high strength and flexibility. For another example, the polyurethane resin for the first polyurethane film layer 10 is a high-strength polyurethane resin A, and the polyurethane resin for the second polyurethane film layer 30 is a high-ductility polyurethane resin C, and they are bonded via the adhesive layer 20 to obtain a polyurethane condom 100 having an ANC layer structure having both high strength and high stretchability. For another example, the polyurethane resin for the first polyurethane film layer 10 is a low-modulus polyurethane resin B, and the polyurethane resin for the second polyurethane film layer 30 is a high burst-pressure polyurethane resin E, and they are bonded via the adhesive layer 20 to obtain a polyurethane condom 100 having a BNE layer structure having both high strength and a high burst pressure. The present disclosure utilizes the adhesion of the adhesive layer 20 to exert the performance advantages of each of the first polyurethane film layer 10 and the second polyurethane film layer 30, so as to obtain a polyurethane condom with an optimized performance. In addition, for example, the second polyurethane film layer 30 may have a thickness different from that of the first polyurethane film layer 10. For example, the first polyurethane film layer 10 may be 10 µm thick while the second polyurethane film layer 30 may be 15 µm thick; or the first polyurethane film layer 10 may be 15 µm thick while the second polyurethane film layer 30 may be 10 µm thick. The present invention is certainly not limited thereto

In other embodiments, the second film layer 30 is, for example, a natural rubber or a polyisoprene resin. In some embodiments, the second film layer has a 100% modulus of, for example, 1 N/mm² or lower, preferably, 0.7 N/mm² or lower, so as to have excellent flexibility. Thus, the polyurethane film layer and a natural rubber or polyisoprene resin film layer can be tightly bonded via the adhesive layer 20, so that the condom prepared has both the properties of the polyurethane resin and the properties of the natural rubber or polyisoprene resin.

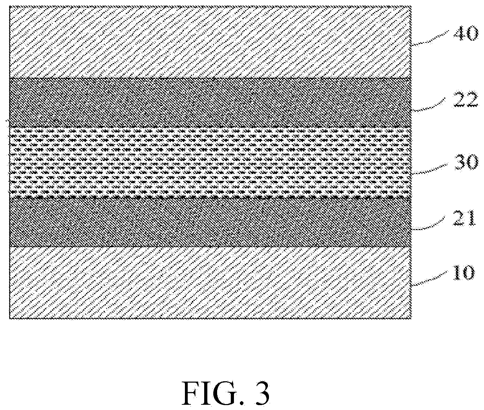

As shown in FIG. 3, the polyurethane condom 100 having an adhesive layer may be formed to have more layers in the structure. Specifically, it may be selected according to the material for the second film layer 30.

As shown in Fig. 3, the present disclosure also discloses another specific embodiment of the polyurethane condom 100 composed of a polyurethane material and a natural rubber or polyisoprene, comprising a first polyurethane film layer 10, a first adhesive layer 21, a second film layer 30, a second adhesive layer 22, and a third polyurethane film layer 40.

As shown in Fig. 3, the adhesive layers include a first adhesive layer 21 and a second adhesive layer 22, which are, for example, composed of the same resin. On the opposite sides of the first adhesive layer 21, the first polyurethane film layer 10 and the second film layer 30 are respectively laminated, and on the opposite sides of the second adhesive layer 22, the second film layer 30 and the third film layer 40 are respectively laminated.

As shown in Fig. 3, the first polyurethane film layer 10, the first adhesive layer 21, the second film layer 30, and the second adhesive layer 22 may have structures, for example, the same as those described above. The third film layer 40 is on the second film layer 20 and has the same structure as that of the first polyurethane film layer 10. In a specific embodiment, for example, the polyurethane condom 100 has a thickness of 0.010-0.080 mm and shows no delamination when immersed in an alkaline solution at 50°C-70°C for 1-10 min. In addition, the first polyurethane film layer 10 and the third polyurethane film layer 40 may have the same structure, and have a tensile strength of 25 MPa or more, such as 35 MPa or more. The adhesive layers 21, 22 are formed of an aliphatic sulfonic acid-based waterborne polyurethane resin having a molecular weight of 30,000 g/mol and a 100% tensile modulus of 2.5 N/mm² or lower, such as 1.8 N/mm² or lower. The second film layer 30 may be a polyisoprene resin having a 100% tensile modulus of 1 N/mm² or lower, such as 0.8 N/mm² or lower. The present invention is certainly not limited thereto, and more layers may be formed, for example, a three-layer structure of a natural rubber or polyisoprene resin layer + an adhesive layer + a polyurethane film layer, or a five-layer structure of a natural rubber or polyisoprene resin layer + an adhesive layer + a polyurethane film layer + an adhesive layer + a natural rubber or polyisoprene resin layer, or a five-layer structure of a polyurethane film layer + an adhesive layer + a natural rubber or polyisoprene resin layer + an adhesive layer + a polyurethane film layer, etc. but not limited thereto. It should be understood that any structure in which an adhesive layer 20 is used as an intermediate layer of the polyurethane condom 100 and on the opposite sides thereof film layers are laminated should fall within the scope of the present disclosure.

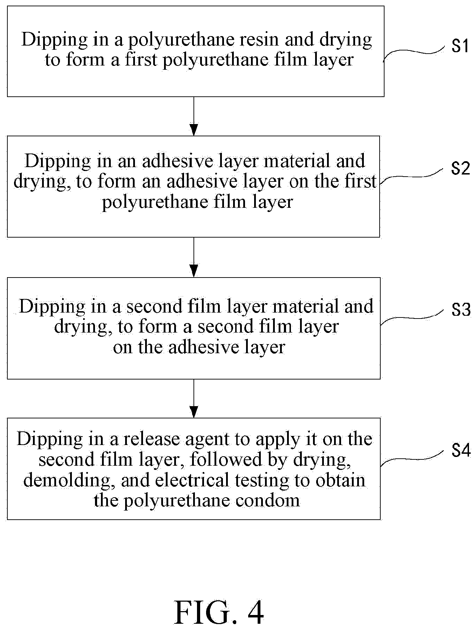

As shown in Fig. 4, the present disclosure also provides a method for preparing the polyurethane condom having an adhesive layer, which comprises, but is not limited to, the following steps S1-S4:
Step S1, dipping in a polyurethane resin and drying, to form a first polyurethane film layer;
Step S2, dipping in an adhesive layer material and drying, to form an adhesive layer on the first polyurethane film layer;
Step S3, dipping in a second film layer material and drying, to form a second film layer on the adhesive layer;
Step S4, dipping in a release agent to apply it on the second film layer, followed by drying, demolding, and electrical testing to obtain the polyurethane condom having an adhesive layer.

During steps S1-S4 as described above, for example, a glass mould may be used and the surface of the glass mould is heated to 20°C to 50°C, e.g. 35°C or 40°C; dipped in the first polyurethane resin and dried at for example 80°C to 120°C, e.g. 90°C, 100°C, or 110°C; dipped in the adhesive layer material and dried at 70°C to 110°C, e.g. 80° C, 90° C, or 100° C; and dipped in the second film layer material and dried at 100°C to 120°C, e.g. 110°C; after rolling, the polyurethane condom is dried at 100°C to 150°C, e.g. 120°C, 135°C or 140°C, then dipped in the release agent and dried at 75°C to 95°C, e.g. 78°C, 80°C or 85°C, followed by demoulding, electrical testing, inner packaging, and outer packaging to obtain the polyurethane condom product.

The present disclosure is further described in detail by using the examples below.

### EXAMPLE

### Example 1

The raw materials for the composite polyurethane condom included:
Polyurethane Resin (having a modulus of 2.5 N/mm² and tensile strength of 55 MPa),
Aliphatic sulfonic acid-based waterborne polyurethane resin for the adhesive layer (having a modulus of 1.0 N/mm² and tensile strength of 5 MPa),
Polyisoprene (PI) Resin.

A glass mould was cleaned, heated to a surface temperature of 30°C, dipped in the polyurethane resin to a thickness controlled at 0.013 mm, and oven-dried at 90°C. Then the resultant was dipped in the adhesive layer material to a thickness controlled at 0.002 mm, and oven-dried at 80°C. Then the resultant was dipped in the polyisoprene resin to a thickness controlled at 0.015 mm and oven-dried at 100°C. After rolling, the product was oven-dried at 120 °C, dipped in a release agent, and oven-dried at 80°C, followed by demoulding, electrical testing, inner packaging, and outer packaging to prepare Polyurethane Condom 1 having a structure of a polyurethane film layer + a polyurethane film layer + a polyisoprene film layer. Upon testing, Polyurethane Condom 1 had a thickness of 0.03 mm, a 100% modulus of 0.8 N/mm², a burst pressure of 2.0 kPa, a burst volume of 25 L, and a tensile strength of 40 N/mm², and showed no delamination when immersed in a NaOH solution at 50°C for 1 min.

### Example 2

The raw materials for the composite polyurethane condom included:
Polyurethane Resin (having a modulus of 2.0 N/mm² and tensile strength of 50 MPa),
Aliphatic sulfonic acid-based waterborne polyurethane resin for the adhesive layer (having a modulus of 0.8 N/mm² and tensile strength of 3 MPa),
Polyisoprene (PI) Resin.

A glass mould was cleaned, heated to a surface temperature of 40°C, dipped in a natural rubber to a thickness controlled at 0.010 mm, and oven-dried at 110°C. Then the resultant was dipped in N to a thickness controlled at 0.003 mm, and oven-dried at 100°C. Then the resultant was dipped in the polyurethane resin to a thickness controlled at 0.010 mm and oven-dried at 110°C. Then the resultant was dipped in N to a thickness controlled at 0.003 mm, and oven-dried at 100°C. Then the resultant was dipped in a natural rubber to a thickness controlled at 0.010 mm and oven-dried at 120°C. After rolling, the product was oven-dried at 140°C, dipped in a release agent, and oven-dried at 80°C, followed by demoulding, electrical testing, inner packaging, and outer packaging to prepare Polyurethane Condom 2 having a structure of a natural rubber layer + an adhesive layer + a polyurethane film layer + an adhesive layer + a natural rubber layer. Upon testing, Polyurethane Condom 2 had a thickness of 0.036 mm, a 100% modulus of 0.8 N/mm², a burst pressure of 2.0 kPa, a burst volume of 30 L, and a tensile strength of 40 N/mm², and showed no delamination when immersed in a NaHCO₃ solution at 60°C for 3 mins.

### Example 3

The raw materials for the composite polyurethane condom included:
Polyurethane Resin (having a modulus of 1.4 N/mm² and tensile strength of 40 MPa),
Aliphatic sulfonic acid-based waterborne polyurethane resin for the adhesive layer (having a modulus of 1.0 N/mm² and tensile strength of 5 MPa),
Polyisoprene (PI) Resin.

A glass mould was cleaned, heated to a surface temperature of 40°C, dipped in the polyurethane resin to a thickness controlled at 0.013 mm, and oven-dried at 110°C. Then the resultant was dipped in N to a thickness controlled at 0.002 mm, and oven-dried at 100°C. Then the resultant was dipped in the polyisoprene resin to a thickness controlled at 0.010 mm and oven-dried at 110°C. Then the resultant was dipped in N to a thickness controlled at 0.002 mm, and oven-dried at 100°C. Then the resultant was dipped in the polyurethane resin to a thickness controlled at 0.013 mm and oven-dried at 120°C. Then the resultant was dipped in a release agent, and oven-dried at 80°C, followed by demoulding, electrical testing, inner packaging, and outer packaging to prepare Polyurethane Condom 3 having a structure of a polyurethane film layer + an adhesive layer + a polyisoprene film layer + an adhesive layer + a polyurethane film layer. Upon testing, Polyurethane Condom 3 had a thickness of 0.04 mm, a 100% modulus of 1.0 N/mm², a burst pressure of 2.6 kPa, a burst volume of 20 L, and a tensile strength of 52 N/mm², and showed no delamination when immersed in a KOH solution at 60°C for 5 min.

The above description is merely preferred embodiments of the present disclosure, and is not intended to limit the present disclosure. For a person skilled in the art, the features in the above-described embodiments can be combined with each other as long as there is no conflict, and the present disclosure can also have various modifications and changes. Any modifications, equivalents, improvements, etc. that fall within the spirit and principles of the present disclosure are intended to be included within the scope of the present disclosure. Furthermore, the examples are to be construed as illustrative and not restrictive, while the scope of the present disclosure is defined by the appended claims rather than by the foregoing description. All changes which come within the meaning and range of equivalency of the claims are therefore intended to be included herein. Any reference number used in a claim should not be construed as limiting the claim.

## Claims

1. A polyurethane condom having an adhesive layer, comprising at least
a first polyurethane film layer;
an adhesive layer on the first polyurethane film layer; and
a second film layer on the adhesive layer,
wherein the adhesive layer includes 0 to 100% by weight of a polar component, 0 to 100% by weight of a non-polar component, and 0 to 100% by weight of a material composed of a polar and non-polar hybrid component, and has a thickness of 0.1 to 30 µm.

2. The polyurethane condom having an adhesive layer according to claim 1, having a burst pressure of 1 kPa or more, and/or a burst volume of 5 L or more.

3. The polyurethane condom having an adhesive layer according to claim 1, having a thickness of 0.010 to 0.080 mm.

4. The polyurethane condom having an adhesive layer according to claim 1, wherein the adhesive layer has a 100% tensile modulus of 2.5 N/mm² or lower.

5. The polyurethane condom having an adhesive layer according to claim 1, wherein the polar component is any one selected from polyurethane resins, neoprene, polyvinyl acetate resins, acrylonitrile-butadiene-styrene, vinyl acetate-ethylene copolymer resins, polyacrylates, polyvinyl chlorides, proteins, starches, celluloses, and epoxy resins, or any combination thereof.

6. The polyurethane condom having an adhesive layer according to any one of claims 1-5, wherein the adhesive layer includes 100% by weight of the polar component which is a polyurethane resin.

7. The polyurethane condom having an adhesive layer according to claim 6, wherein the polyurethane resin is any one selected from anionic, cationic, nonionic, and amphoteric waterborne polyurethanes, or solvent-based and solvent-free polyurethanes, or any combination thereof.

8. The polyurethane condom having an adhesive layer according to claim 7, wherein the polyurethane resin is any one of polyether-based, polyester-based, and polyether-polyester hybrid polyurethane resins, or any combination thereof.

9. The polyurethane condom having an adhesive layer according to claim 7, wherein the anionic waterborne polyurethane is a carboxylic acid-based polyurethane, a sulfonic acid-based polyurethane, or a mixture of both.

10. The polyurethane condom having an adhesive layer according to claim 1, wherein the non-polar component is one selected from natural rubbers, polyolefins, silicone resins, butyl rubbers, styrene-butadiene rubbers, ethylene-propylene-diene rubbers, polystyrenes, polyisoprenes, polyesters, rosin resins, terpene resins, rosin resins and stretched forms thereof, or any combination thereof.

11. The polyurethane condom having an adhesive layer according to claim 1, wherein the polar and non-polar hybrid component is selected from an organically modified polar or non-polar polymeric material.

12. The polyurethane condom having an adhesive layer according to claim 11, wherein the organic modification is any one selected from organosilicon modification, organofluorine modification, silane modification, acrylate copolymerization modification, vinyl modification, aromatic monomer modification, polyether modification, polyester modification, isocyanate modification, amination, carboxylation, hydroxylation, epoxidation, sulfonation, and polymerization modification, or any combination thereof.

13. The polyurethane condom having an adhesive layer according to claim 1, wherein the adhesive layer includes 50% by weight of a polar component and 50% by weight of a non-polar component.

14. The polyurethane condom having an adhesive layer according to claim 1, comprising at least one adhesive layer.

15. The polyurethane condom having an adhesive layer according to claim 1, wherein the second film layer has a 100% modulus of 1 N/mm² or lower.

16. The polyurethane condom having an adhesive layer according to claim 1 or 15, wherein the second film layer is a layer composed of a polyurethane resin.

17. The polyurethane condom having an adhesive layer according to claim 1, wherein the second film layer is a layer composed of a natural rubber or a polyisoprene resin.

18. A polyurethane condom having an adhesive layer, comprising:
a first polyurethane film layer;
a first adhesive layer on the first polyurethane film layer;
a second film layer on the adhesive layer;
a second adhesive layer on the second film layer, the second adhesive layer having the same structure as that of the first adhesive layer; and
a third polyurethane film layer on the second film layer, the third polyurethane film layer having the same structure as that of the first polyurethane film layer,
wherein the first adhesive layer includes 0 to 100% by weight of a polar component, 0 to 100% by weight of a non-polar component, and 0 to 100% by weight of a material composed of polar and non-polar hybrid component, and has a thickness of 0.1-30 µm;
the first adhesive layer has a 100% tensile modulus of 2.5 N/mm² or lower;
the second film layer includes a natural rubber or a polyisoprene resin; and
the polyurethane condom has a thickness of 0.010-0.080 mm.

19. A method for preparing a polyurethane condom having an adhesive layer, comprising at least the steps of:
dipping in a polyurethane resin and drying, to form a first polyurethane film layer;
dipping in an adhesive layer material and drying, to form an adhesive layer on the first polyurethane film layer;
dipping in a second film layer material and drying, to form a second film layer on the adhesive layer;
dipping in a release agent to apply it on the second film layer, followed by drying, demolding, and electrical testing to obtain the polyurethane condom having an adhesive layer;
wherein the adhesive layer includes 0 to 100% by weight of a polar component, 0 to 100% by weight of a non-polar component, and 0 to 100% by weight of a material composed of polar and non-polar hybrid component, and has a thickness of 0.1-30 µm.

20. The method for preparing a polyurethane condom having an adhesive layer according to claim 19, wherein the dipping in a polyurethane resin is carried out by dipping a mould composed of glass or a thermoplastic polymer in the polyurethane resin.
